# EUROPÄISCHE PATENTANMELDUNG

(11) **EP 2 418 474 A1**
(43) Veröffentlichungstag der Anmeldung: **15.02.2012**
(21) Anmeldenummer: 10171621.5
(22) Anmeldetag: 02.08.2010
(51) Int. Cl.: G01N 21/35, G01J 3/28, G06F 19/00, G01N 15/02, G01N 33/04

(54) **Nahinfrarotspektroskopieverfahren zur gleichzeitigen Ermittlung des Komponentengehalts in der Rohmilch**

(71) Anmelder: OOO "Novye Energeticheskie Technologii", Moskau 107045 (RU); "Golsen Limited" Georgiou GENLNLADLIOU, Limassol 3041 (RU)
(72) Erfinder: Kalinin, Andrey V., Troitsk 142092 (RU)
(74) Vertreter: Jeck, Anton

(57) **Zusammenfassung**

Beschrieben wird ein Nahinfrarotspektroskopieverfahren (NIRS-Verfahren) zur gleichzeitigen Ermittlung des Gehalts der Komponenten (Fett, Protein, Wasser, Körperzellen, Bakterien und anderer Komponenten) in Milch einschließlich
- der Suche nach einem Satz der Milchproben mit bekanntem Gehalt der zu ermittelnden Komponenten (dem sog. Kalibriersatz),
- der Speicherung der NIRS-Spektren der Wechselwirkung der o. g. Proben mit der darauf einfallenden Strahlung,
- der Ermittlung des Regressions-(Kalibrierungs)-Modells zwischen den gespeicherten (gesammelten) Spektren und den bekannten Gehaltswerten der zu ermittelnden Komponenten,
- der Messung der NIRS-Spektren der Wechselwirkung der Testproben,
- der Prüfung des o. g. Kalibrierungsmodells anhand dieser NIRS-Spektren der Proben,
- der Messung von NIRS-Spektren der Wechselwirkung der zu untersuchenden Proben und
- der Ermittlung des Komponentengehalts in der zu untersuchenden Milch nach dem Kalibrierungsmodell unter Berücksichtigung der Prüfungsergebnisse, wobei

die Kalibriercharge von Milch mit unkorrelierenden Variationen des Fettgehalts und der größengerechten Verteilungsparameter der Fettkügelchen und anderer Milchteilchen ausgesucht und das Kalibrierungsmodell unter Beachtung der Variation der Verteilungsparameter je nach Teilchengröße der Milch ermittelt wird, um die Genauigkeit und die Zuverlässigkeit bei der Gehaltsermittlung von Milchkomponenten zu erhöhen;

## Beschreibung

### Gebiet der Technik

Die Erfindung ist für die Anwendung in technischen Einrichtungen zur Kontrolle der physikalisch-chemischen und biologischen Gütezahlen der unbehandelten Rohmilch vorgesehen. Diese Einrichtungen können unter anderem in die Melkapparate und Milchleitungen eingebaut werden. Die Erfindung kann auch für die wirtschaftlichen Berechnungen und Selektionsarbeiten in Landwirtschaftsbetrieben und Milchhäusern sowie in Milchsammeistellen angewendet werden.

### Stand der Technik, Beschreibung der Vergleichserzeugnisse und des Phototyps

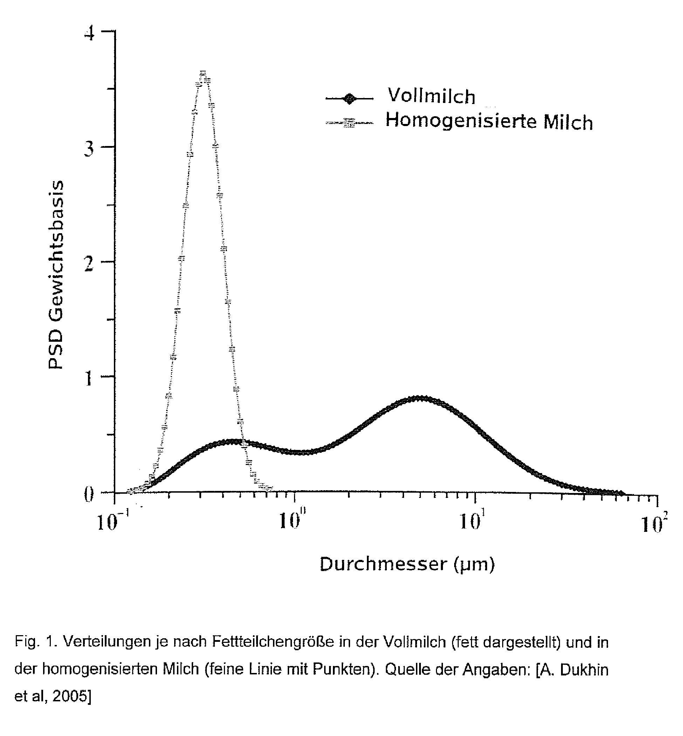

Milch ist eine kolloidale Mehrkomponentendispersion. Sie enthält Fettkügelchen, Kaseinmizellen, Molkenproteinaggregate, Körperzellen und Bakterien als disperse Phasen und Wasserlösungen von Salzen, Fermenten und Vitaminen als disperses Medium. Die Rohmilch zeichnet sich durch wesentliche Variationen der Fettkügelchengrößen sowie durch das Vorhandensein von milchspezifischen Bakterien aus. Die durchschnittliche Fettkügelchengröße sowie ihre Dispersionsverteilung je nach Größe variiert im Bereich von 0,1 bis 10 und sogar darüber (s. Fig. 1 aus dem Schriftstück [A. Dukhin et al, 2005]. Sie hängt von vielen Faktoren der Tierzuchttechnik ab [G.V. Tverdokhleb, R.I. Ramanauskas, 2006.]).

Die Anwendung der Nahinfrarotspektroskopie (NIRS) der durch die Milch gestreuten oder aufgenommenen Strahlung in Kombination mit dem statistischen Eichverfahren mit PLS (Projektionen auf latente Strukturen)- Regression zur quantitativen Schnellanalyse der Rohmilch ist allgemein bekannt. Dabei können unter anderem tragbare Spektrometer eingesetzt werden, die in die Produktionslinie integriert sind [s. Beschreibung des Stands der Technik zum Patent RU 92191, A.V. Kalinin, 2009, und das Schriftstück von Tzenkova R., et al., 2006].

Die NIRS-Spektren von Milch enthalten Angaben sowohl über die Anteile der chemischen Komponenten: Wasser, Fette, Proteine, Milchzucker, Salze, Vitamine, Fermente als auch über die physikalischen Kennwerte der dispersen Phasen: Dispersitätsgrad, Größe und Struktur der Teilchen. Das Signal des NIRS-Spektrometers wird durch die Abschwächung der auf die Probe einfallenden Strahlung erzeugt. Diese Abschwächung ist sowohl durch die Streuung mittels Teilchen als auch durch die Aufnahme der Strahlung durch den Stoff des Mediums und der Teilchen bedingt. Dabei ist der Streufaktor von Milch im NIRS-Bereich zehn- bis hundertmal höher als der Absorptionsgrad. Bekanntlich [s. Walstra, P. 1965. Anderson M. E., et al, 1985] sind die instabile Kalibrierung und die nicht zufriedenstellende Genauigkeit der oben genannten Verfahren auf die zu komplizierte Größenverteilung der dispersen Milchphasen sowie auf ihre Variationen je nach unterschiedlichen Faktoren der Tierzuchttechnik zurückzuführen. Das wurde auch im Schriftstück [A.V. Kalinin et al., 2009] beschrieben.

Die Beschreibung zum Patent EP 1 444 501 B1 vom 08.11.2001 offenbart den spektroskopischen Milch-analysator AfiLab der Fa. AfiMilk, Israel. Er beruht auf dem Verfahren zur Messung der Spektren im Kurzwellen-bereich bei NIRS (KW-NIRS) beim Durchlassen und bei der Streuung unter verschiedenen fest eingestellten Winkeln. Es handelt sich dabei um ein ziemlich preiswertes jedoch ungenügend präzises Einbaugerät, das bei der Bestimmung der Milchproteine besonders ungenau ist.

Die KW-NIR-Spektrometrie wurde vor kurzem in Kombination mit den statistischen Kalibrierungsverfahren (darunter mit der PLS-Regression) zur Entwicklung der Mittel für die quantitative Schnellanalyse der Rohmilch angewendet [R. Muniz et al, 2009]. In diesem Schriftstück wird ebenfalls auf die Schwierigkeiten bei der Bestimmung des Proteinanteils hingewiesen.

Im Schriftstück [Saranwong S. and Kawano S., 2008] wurde eine Methode zur Ermittlung der Hauptkomponenten der Rohmilch wie Fett und Protein sowie der Gesamtzahl der aeroben Bakterien entwickelt. Dabei wurden das Forschungs-Spektralfotometer NIRSystem 6500, NIRSystems, Silver Spring, MD, und das transportable KW-NIRS-Spektralfotometer FT20, Fantec, Japan, eingesetzt. Es wurden die Kennwerte der Kalibrierungsregressionsmodelle verglichen und die Betriebsart für die beste Bestimmung der oben genannten Komponenten gewählt. Es wurde auch auf die Wichtigkeit der Ermittlung des Protein- und Milchzuckergehalts hingewiesen, um die Keimzahl einschätzen zu können.

Die in oben aufgeführten Schriften genannten Schwierigkeiten bei der Ermittlung der Proteine werden dadurch erklärt, dass die Proteine keine KW-NIRS-Strahlung aufnehmen und im KW-NIRS-Bereich erfasst werden und zwar nur dadurch, dass sie durch die Kaseinmizellen im Hintergrund der um einige Größenordnungen größeren Streuung durch die Fettkügelchen gestreut werden. Die Streuungsvariationen durch die Fettkügelchen sind in der unbehan-delten Rohmilch ziemlich groß. [Walstra, 1965]. Deswegen muss ein so weiter Dynamikbereich des Spektrometers zur Aussonderung der mit der Gehaltsänderung der Kaseinmizellen zusammenhängenden Spektrenvariationen verwendet werden, welcher in den transportablen KW-NIRS-Geräten nicht realisierbar ist.

Um den Einfluss der Verteilungsvariation je nach Teilchengröße auf die optischen Kenndaten der dispersen Materialien zu berücksichtigen, wurden in der letzten Zeit Verfahren zur Spektraltrennung der Streu- und Absorptionsfaktoren auf Grund der Strahlungstransporttheorie und der Mieschen Streuungstheorie (Streuung durch die Teilchen) ausgeführt. So wurde z. B. das bekannte Transmissions- und Streuungsspektrometrie-Verfahren unter Anwendung der sog. Integrierkugeln entwickelt, um die KW-NIRS-Spektren der optischen Milchkennwerte zu ermitteln [I.V. Yaroslavsky et. al., 1996]. In der Kombination mit dem Regressionskalibrierungsverfahren wurde dieses Verfahren zur quantitativen Analyse der Milchkomponenten, darunter auch der Proteine, verwendet [s. Z. He, et al., 2005]. Jedoch war die spektroskopische Rückaufgabe inkorrekt, bzw. es gab keine allgemeine Lösung für diese Aufgabe in Bezug auf solche konzentrierten Dispersionen wie z. B. Milch. Deshalb ergeben die in den o. g. Verfahren benutzten Approximationen bei der quantitativen Milchanalyse keine eindeutigen Ergebnisse und sind somit für den praktischen Einsatz nicht geeignet.

Aus dem US-Patent [X.H.Hu, 2009] ist des Weiteren das Verfahren zur Bestimmung der optischen Kennwerte der trüben Stoffe, darunter auch Milch, bekannt. Bei diesem Verfahren werden die Spektren der Streu- und Absorp-tionsfaktoren und des Anisotropiefaktors der Probe bestimmt (die Probe wird dabei vor allem durch das Verhältnis der Teilchengröße zur Strahlungswellenlänge festgelegt). Dabei wurde das Mehrkanalabtastspektrometer im Licht- und NIRS-Bereich eingesetzt. Jedoch kann dieses Verfahren zur quantitativen Schnellanalyse der Milch wegen der platzraubenden Konstruktion, des zu hohen Preises und der zeitraubenden Spektralmessung nicht angewendet werden.

Der nächste Stand der Technik gegenüber der angemeldeten Erfindung ist das im Schriftstück [A.V. Kalinin, et al., 2008] beschriebene NIRS-Verfahren zur Feuchtebestimmung in Pulvermilch. Nach diesem Verfahren wird ein Regressionskalibrierungsmodell anhand der Feuchtewerte gebaut. Die Feuchteangaben werden dabei mithilfe des thermogravimetrischen Feuchteanalysators (TGA) ermittelt. Um den Einfluss der Änderung des physikalischen Parameters ― nämlich der Probetemperatur― auf die NIRS-Spektren und das Kalibrierungsmodell auszuschließen, wird eine zusätzliche Operation und zwar die Temperaturmessung der Pulvermilchprobe durch den in den TGA eingebauten Messwertgeber durchgeführt. Darüber hinaus werden noch die Mehrstufenerwärmung der Probe während der Messungen ihrer variablen Feuchte und die Erfassung der NIRS-Spektren der diffusen Reflexion vorgenommen. Dank der Anwendung der Mehrstufenerwärmung wurden unkorrelierende Feuchte-und Temperaturwerte der Proben erhalten und dazu noch gleichzeitig ihre NIRS-Spektren erfasst. Anhand dieser Angaben wurde aufgrund des Verfahrens der PLS-Regression das Kalibrierungsmodell gebaut. Mit Hilfe dieses Modells wurden die Feuchtewerte der Proben bei ihrer beliebigen Temperatur innerhalb des vorgegebenen Bereichs bestimmt.
Die vorliegende Erfindung verwirklicht das gleiche Vorgehen jedoch unter Einsatz von anderen Mitteln und in Bezug auf die anderen zu ermittelnden Milchkennwerte.

### Das Wesen der Erfindung

Es sei bemerkt, dass einige Druckschriften eine einzigartige Eigenschaft von Milch feststellen - den Korrelationszusammenhang zwischen der durchschnittlichen Kugelgröße rₘ und dem Fettgehalt. Dieses wurde auch in der Druckschrift [H.W. Jensen et al., 2007] zusammengefasst. Diese Eigenschaft wird für die Gesamtfettwerte in der Rohmilch von 2%wt>F>6,5%wt durch die Funktion F ∼ a rₘ³ beschrieben, wobei a im Bereich von 0,4 bis 1,2 je nach Faktoren der Tierzuchttechnik liegt. Das heißt, der Fettgehalt in der Rohmilch variiert proportional zum durchschnittlichen Kugelumfang. Die Dichte der Kugelzahl ändert sich nur sehr wenig mit der Fettgehaltänderung innerhalb des angegebenen Bereichs. Folglich können einerseits die mit der Milchverdünnung verbundenen Kalibrierungsverfahren für die NIR-Spektrometrie von Milch nicht angewendet werden. Andererseits muss vor allem der Einfluss der größenmäßigen Verteilungsvariation der Kugeln auf den Streuungsverlauf und die Abschwächung der Strahlung und folglich auf die Kalibrierung des NIRS-Spektrometers und die Dichtevariationen der Zerstreuungsteilchen berücksichtigt werden.

Die Ziele der vorliegenden Erfindung sind die Erhöhung der Genauigkeit und der Zuverlässigkeit sowie die Beschleunigung und Verbesserung des Ablaufs des NIRS-Verfahrens zur Gehaltsermittlung von Fetten, Proteinen, Milchzucker, Wasser, Körperzellen und Bakterien in der Frischmilch, die keiner ihre physikalischchemische Struktur verändernden Behandlung ausgesetzt wird, und dementsprechend die Entwicklung von einem Verfahren, welches für die Anwendung in den in die Produktionslinien integrierten Geräten geeignet ist. Das weitere Ziel der Erfindung ist die Senkung der Kalibrierungskosten bei einer der Ausführungsformen des Verfahrens.

Das technische Ergebnis der Anwendung der vorliegenden Erfindung kann die sichere und genaue Ermittlung der oben genannten Milchkomponenten im Rahmen der gültigen "Technischen Vorschriften für Milch und Milchwaren" sowie gemäß GOST R 25054-2003 Natürliche Kuhmilch ― Rohstoffe", darunter auch mit Hilfe von den in die Produktionslinie eingebauten KW-NIRS Spektrometern, sein.

Das wesentliche Merkmal der vorliegenden Erfindung und ihres am nächsten liegenden Stands der Technik ist folgender Ablauf der auszuführenden Operationen:
a) Suche nach einem Satz der Milchproben (zur Kalibrierung) mit dem auf eine spezifische Weise vorgegebenen Gehalt an zu ermittelnden Komponenten und den physikalischen Kennwerten, welche die Spektren der Zusammenwirkung der NIRS-Strahlung mit dem Material der Probe beeinflussen, wobei die Variationen der Gehalts- und physikalischen Kennwerte gegenseitig nicht korrelierend sind,
b) Speicherung der NIRS-Spektren der Wechselwirkung der o .g. Proben mit der darauf einfallenden Strahlung,
c) Ermittlung und Prüfung des Regressions-(Kalibrierungs)-Modells nach den gespeicherten NIRS-Spektren und den bekannten Gewichtsanteilen der zu ermittelnden Komponenten und der o. g. physikalischen Kennwerte der Kalibrierungs- und der Testgruppe der Proben,
d) Messung der NIRS-Spektren der zu untersuchenden Milch und die Verwendung des Kalibrierungsmodells zur Lösung der spektrometrischen Umkehraufgabe und zwar die Ermittlung des Komponentengehalts in der zu untersuchenden Milch nach ihren NIRS-Spektren.

Das gesetzte Ziel ― die Erhöhung der Genauigkeit und der Zuverlässigkeit bei der Gehaltsermittlung nach vorliegendem Verfahren wird folgenderweise erreicht.
Es wird eine Kalibriercharge von Milch mit der Verteilungsvariation je nach Kugelgrößen ausgesucht die mit der Variation des Gesamtfetts nicht korreliert. Danach wird eines der nachfolgenden Verfahren ausgeführt:
a) Auswahl der Proben mit unkorrelierenden Variationen des Gesamtfetts und der durchschnittlichen Kugelgröße als Kalibriersatz. Solche Proben werden aus einer ziemlich großen Menge der Milchproben ausgewählt. Dabei wird die durchschnittliche Kugelgröße nach einem der bekannten Verfahren gemessen: Laserbeugung, konfokale Mikroskopie oder Akustisches Verfahren [R. Attaie and R. L. Richter†, 2001; M-C. Michalski, et al., 2003; A. Dukhin et al, 2005].
b) Einige sich dem Gesamtfett nach sehr unterscheidenden Rohmilchproben werden in einige Fraktionen je nach durchschnittlichen Kugelgrößen mittels Mikrofiltration oder Schwerkraftabsetzens oder Zentrifugierens und einer Homogenisierung aufgeteilt. Danach wird ein Kalibriersatz gefertigt, indem diese Fraktionen mit Rückgabemilch (Magermilch) im vorgegebenen Verhältnis gemischt werden. Dabei müssen die durchschnittlichen Kugelgrößen in den zubereiteten Gemischen bei zwei letzten Verfahrensschritten der Fraktionierung nach einem selbständigen Verfahren gemessen werden, wie es im Unterpunkt a) des Absatzes [016] angegeben ist; z. B. mittels eines Geräts für die Laserbeugung Mastersizer 2000, Malvern Co, UK. Es ist auch die Anwendung anderer Geräte möglich;
   - danach wird das Kalibrierungsmodell gebaut (ermittelt), z. B. aufgrund des Verfahrens der PLS-Regression, darunter auch unter Anwendung der durchschnittlichen Kugelgrößen.

Das gesetzte Ziel der Beschleunigung und Verbesserung des Ablaufs wird nach vorliegendem Verfahren folgenderweise erreicht.
Es werden zusätzlich die NIRS-Spektren der Richtungsabhängigkeit der Streuung der Proben aus dem Kalibriersatz und der zu untersuchenden Milch gemessen. Danach werden die Spektral- und Richtungsabhängigkeiten der Streuung der Proben aus dem Kalibriersatz nach einem bekannten Verfahren [z.B. I.V. Yaroslavsky, et al., 1996] auf Basis der Strahlungstransporttheorie und der Mie-Theorie berechnet. Anhand dieser Spektral- und Richtungsabhängigkeiten wird die durchschnittliche Kugelgröße in den Proben bewertet. Die Proben für den Kalibriersatz werden gemäß der Beschreibung aus a), Absatz [016], ausgewählt.

Das gesetzte Ziel der Senkung der Kalibrierungskosten nach vorliegendem Verfahren wird folgenderweise erreicht.
Die Speicherung der NIRS-Spektren der Wechselwirkung der o. g. Proben mit der darauf einfallenden Strahlung erfolgt mittels Berechnung der Spektralverläufe der Strahlungsstreuung und -absorption sowie der Simulation der Spektren des NIRS-Spektrometers nach einem bekannten Verfahren [S.N. Thennadil and E.B. Martin, 2005] für vorgegebene Variationen des Komponentengehalts und der größenbedingten Teilchenverteilungen, um danach anhand dieser Angaben das Kalibrierungsmodell zu bauen.

### Angaben, die die Ausführbarkeit des Verfahrens nachweisen

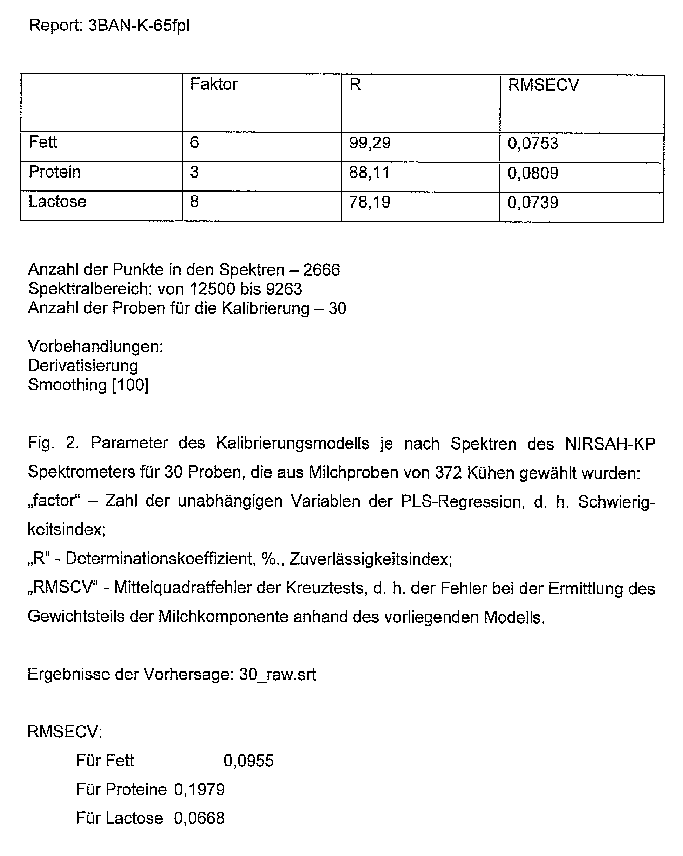

Das Ergebnis einer der möglichen Ausführungsformen des Verfahrens ist in Fig. 2 abgebildet. Der Kalibriersatz enthält 30 Proben der unhomogenisierten Rohmilch. Der Kalibriersatz wurde aus Einzelproben von 372 Kühen von 3 Viehzuchtfarmen des Instituts für Viehzucht der Russischen Akademie für landwirtschaftliche Wissenschaften, Dubrovitsy, Podolsky Rayon, Moskauer Gebiet so ausgewählt, dass die Variation des Gesamtfett s im Satz 2,6 ― 6,6 % (Gew.) betrug. Die Variation wurde im Labor des Instituts für Viehzucht mit einem Spektrometer "Bently", Bently Instruments Corp., US gemessen. Die Variation der durchschnittlichen Kugelgröße wurde mit dem Spektrometer "Zetasizer", Malvern Co, UK, gemessen und machte 1,2 ― 8,2 µm aus. Dabei betrug der Koeffizient der Paarwerte gegenüber der Korrelation in den Proben 52 %. Die Durchlassspektren und die Spektren der diffusen Streuung im Bereich der KW-NIRS (0,8 ― 1,07 µm) wurden mit dem NIRS-Spektrometer NIRSAH-KP, OOO NET, Moskau, gemessen. Mit Hilfe der Software für die PLS-Regression ISCAP (Entwickler: S.V. Sadovsky, amtliche Eintragungsnr.......) wurde das Kalibrierungsmodell mit einem hohen Sicherheitsgrad gebaut. Der Determinationskoeffizient R bei der Ermittlung von Gesamtfett und Protein betrug jeweils 99,29 % und 88,11 %. Der Mittelquadratfiehler bei der Ermittlung nach dem vorliegenden Verfahren (s. Fig. 2) entspricht den Anforderungen der "Technischen Vorschriften für Milch und Milchwaren" sowie der GOST R 25054-2003 "Natürliche Kuhmilch ― Rohstoffe". Dabei gelang es im vorliegenden Modell, zum ersten Mal den Milchzuckergehalt zu ermitteln. Ansonsten ist dies immer wegen der beachtlichen Variation der Spektren erschwert, was auf die Fett- und Kugelgrößenvariation zurückzuführen ist.

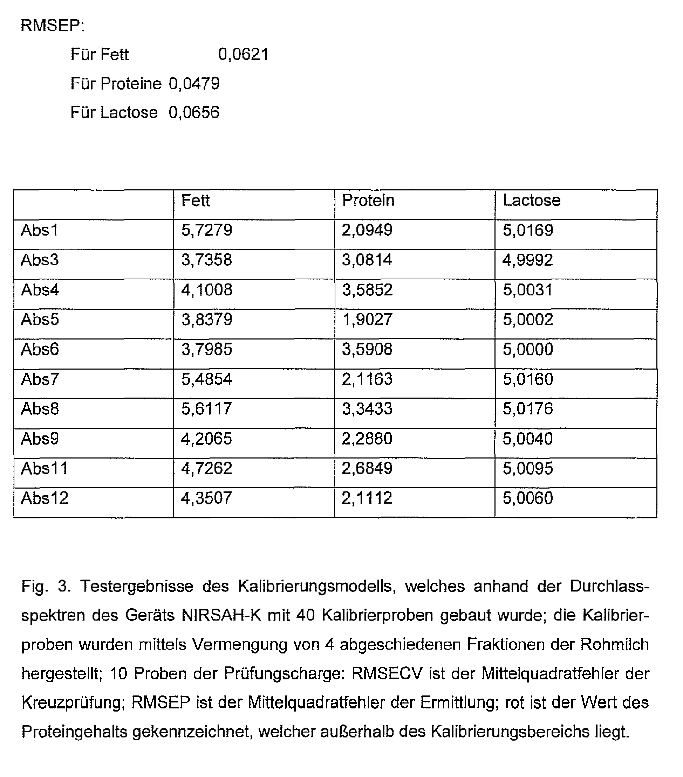

Gemäß einer anderen Ausführungsform des vorliegenden Verfahrens wurde der Kalibriersatz aus 50 Proben hergestellt, indem 4 Fraktionen der Rohmilch gemischt wurden. Sie wurden mittels Zentrifugierens von 3 Rohmilchproben mit einer Fettvariation von 2,6 ― 6,2 % (Gew.) entrahmt (abgeschieden) und in Bezug auf Fett-, Protein-, Milchzucker-, Trockenrückstandgehalt und auf die Menge der Körperzellen mit Hilfe des Spektrometers "Bently", Bently Instruments Corp., US, und in Bezug auf die durchschnittlichen Kugelgrößen mit Hilfe des Laserkorrelationsspektrometers LKS-03, Moskauer Physikalisch-Technisches Institut, Moskau, gemessen. Ein Teil der Fraktionen wurde in der akustischen Homogenisiervorrichtung der Fa. OOO SAPHIR, Moskau, homogenisiert, um die Einspitzenverteilung mit vorgegebener durchschnittlicher Kugelgröße in Übereinstimmung mit dem in der Druckschrift [M. F. Ertugay, et al., 2004] beschriebenen Verfahren zu bekommen. Die Kenndaten der Fraktionen sind in Tabelle 1 aufgeführt. Für die Vermischung der Fraktionen wurden Einwaagen benutzt. Die Einwaagen wurden in einem anwendungsspezifischen Programm CoDes berechnet. Das Programm dient zur Herstellung der Milchmischungen mit unkorrelierenden Werten des Komponentengehalts und den physikalischen Kennwerten. Das Programm ist in der Druckschrift [A.V. Kalinin, V.N. Krasheninnikov, 2008] beschrieben. Danach wurden die Durchlassspektren von 50 Proben der Kalibriermischungen gemessen. Für die Messungen wurde das im genannten Schriftstück beschriebene Spektrometer NIRSAH-K eingesetzt, welches im Institut für Spektroskopie der Russischen Akademie für Wissenschaften, Troitsk, entwickelt wurde. Anhand von 40 Proben wurde das Kalibrierungsmodell gebaut, und 10 Proben wurden zum Testen des Modells benutzt. Die Testparameter sind in Fig. 3. dargestellt. Der Vergleich solcher Parameter wie Schwierigkeit, Zuverlässigkeit und Genauigkeit (s. Parameterdefinitionen in Fig. 2) nach dieser Ausführungsform des Verfahrens mit der vorhergehenden Ausführungsform hat gezeigt, dass die Parameter des Modells, welches anhand der Fraktionsmischungsproben berechnet worden ist, etwas schlechter sind als die Parameter des Modells mit unbehandelten Proben. Sie sind jedoch für die praktische Anwendung geeignet, um so mehr, als die Realisierung des Verfahrens gemäß Absatz [020] einfacher und mehrfach billiger gegenüber der vorher beschriebenen Ausführungsform ist.

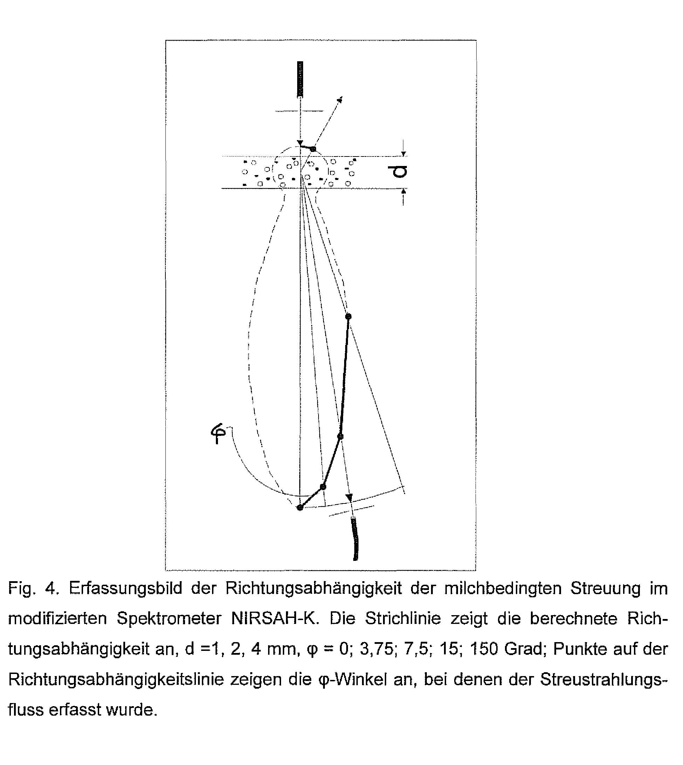

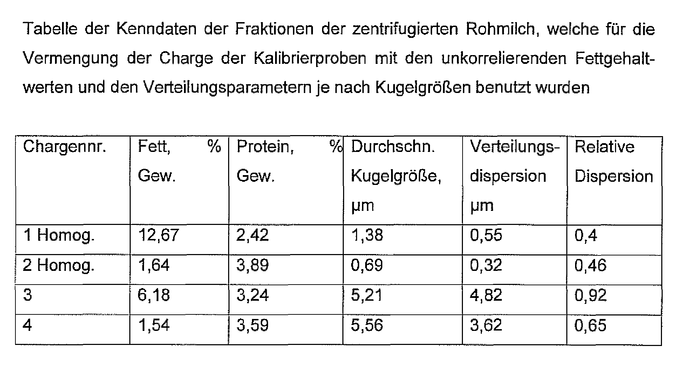

In einer anderen Ausführungsform des vorliegenden Verfahrens wurden zusätzlich die Winkelverteilungen der Streuung der Proben aus dem Kalibriersatz mit 40 Proben, die nach Absatz [020] gefertigt wurden, und 10 Proben der Prüfmilch gemessen. Die Messung erfolgte in einer flachen 1 mm starken Küvette mit Hilfe eines Spektrometers NIRSAH-K, welches dafür modifiziert wurde, den Messwinkel der Streustrahlung zu variieren. Darüber hinaus wurde das Spektrometer mittels eines Lichtfaserkabels mit dem Winkelmesser kombiniert. Dabei wurden die Winkelverteilungen der Streuung für die auf dem Schema in Fig. 4 dargestellte Messgeometrie aufgrund der Kleinwinkel-annäherung der Strahlungstransporttheorie je nach der durchschnittlichen Kugelgröße als Berechnungsparameter berechnet. Der Berechnungsalgorithmus ist in der Druckschrift [N.G. Khlebtsov, 1984] beschrieben. Die Reihenfolge der Berechnungen ist wie folgt: Die Berechnung der Entwicklungskoeffizienten nach dem Legendreschen Polynom der Indikatrix der einzelnen Kugel anhand der analytischen Formeln der Mieschen Streuungstheorie (s. z. B., [A. Ishimary, 1978]); die Indikatrix der (mehrfachen) Streuung durch das Volumenelement von Milch mit vorgegebenem Fettgehalt und größengerechter Kugelverteilung wurde nach der Kleinwinkelannäherug der Lösung der Transportgleichung als die gemäß den Streuungsgraden geordnete Reihe mit Legendresches Polynome und Koeffizienten berechnet, welche nur von der optischen Stärke abhängen, die eindeutig mit dem Volumen der Milchteilchen verbunden ist. Danach wurden die durchschnittlichen Kugelgrößen in den Proben nach der Methode der Anpassung der gemessenen Winkelverteilung an die Rechenwerte bewertet, wie es in der Druckschrift [A.V. Kalinin und V.N. Krasheninnikov, 2010] beschrieben ist. In der gleichen Druckschrift ist das Kalibrierungsmodell dargestellt, welches unter Berücksichtigung der bewerteten durchschnittlichen Kugelgrößen der Milchproben gebaut worden ist. Hier sind auch die Prüfungsergebnisse dargelegt. Aus diesen Ergebnissen kann gefolgert werden, dass die Zuverlässigkeit und die Genauigkeit der Ermittlung von Fett, Protein und Trockensubstanz nach dieser Ausführungsform des Verfahrens denen der Ausführungsform aus Absatz [020] entsprechen. Jedoch erfolgt diese Ermittlung bedeutend schneller als im nächstliegenden Stand der Technik, und zwar dank der Kombination der Spektralmessungen und der Winkelverteilungen in einem Spektrometer NIRSAH-K.

In einer anderen Ausführungsform des vorliegenden Verfahrens wurde der Kalibriersatz der Proben benutzt, bei denen die Fett- und Proteinanteile sowie die durchschnittlichen Kugelgrößen anhand der CoDes-Software gemäß Absatz [020] vorgegeben wurden. Nach dieser Ausführungsform des Verfahrens wurde für diesen Kalibriersatz der Proben die Simulation (die Speicherung) der NIRS-Spektren vorgenommen, indem ihre Spektral- und Winkelverläufe berechnet und die berechneten Spektren mit der vorher gemessenen Hardwarefunktion und dem Rauschspektrum des Spektrometers NIRSAH-K kombiniert wurden. Die Messungen hierfür wurden in Übereinstimmung mit der Beschreibung in der Druckschrift [S.N. Thennadil and E.B. Martin, 2005] durchgeführt. Die simulierten Spektren des Geräts NIRSAH-K und die vorgegebenen Variationen des Komponentengehalts und der größengerechten Verteilung der Milchteilchen wurden benutzt, um die PLS des Kalibrierungsmodells zu bauen. Die Prüfung des Kalibrierungs-modells wurde auf einer Charge mit 12 Rohmilchproben vorgenommen. Diese Proben wurden in Bezug auf Fett- und Proteingehalt im Forschungslabor für Milch des Instituts für die Milchindustrie der Russischen Akademie für landwirtschaftliche Wissenschaften genormt. In Bezug auf die Verteilung je nach Kugelgrößen wurden die Proben mittels des Spektrometers Zetasizer, Malvern Co, UK, geprüft. Ihre Durchlass-NIRS-Spektren wurden mit dem modifizierten Spektrometer NIRSAH-K gemessen. Der Mittelquadratfehler der Vorhersage betrug 0,108 % (Gew.) für Gesamtfett und 0,125 % (Gew.) für den Gesamtproteingehalt.
Da diese Ausführungsform keiner Kosten für die Fertigung der Kalibriercharge und für die Messung ihrer Spektren bedarf, ist sie wirtschaftlich äußerst vorteilhaft, besonders wenn die Anzahl der gleichzeitig zu ermittelnden Komponenten zunimmt.
Die vorliegende Erfindung betrifft also ein Verfahren der Nahinfrarotspektroskopie (NIRS) zur gleichzeitigen Ermittlung des Gehalts der Komponenten der Rohmilch zur Anwendung in den technischen Einrichtungen zur Kontrolle der physikalisch-chemischen und biologischen Gütezahlen der unbehandelten Rohmilch, darunter auch in den Einrichtungen, die in die Melkapparate und Milchleitungen integriert werden. Das Verfahren umfasst folgende Schritte:
a) Suche nach einem Satz der Milchproben (sog. Kalibrierproben) mit unkorrelierenden Variationen des Gehalts der zu ermittelnden Komponenten und der physikalischen Kennwerte, welche die Spektren der Zusammenwirkung der NIRS der Strahlung mit dem Material der Probe beeinflussen,
b) Speicherung der NIRS-Spektren der Wechselwirkung der Kalibrierproben mit der darauf einfallenden Strahlung sowie der Testproben, die den Kalibrierproben ähnlich sind,
c) Ermittlung und Prüfung des Regressions- (Kalibrierungs-) Modells nach den gespeicherten NIRS-Spektren und den bekannten Gewichtsanteilen der zu ermittelnden Komponenten und physikalischen Kennwerten der Kalibrierungs- und der Testgruppe der Proben,
d) Messung der NIRS-Spektren der zu untersuchenden Milch und Verwendung des Kalibrierungsmodells zur Gehaltsermittlung von Komponenten anhand ihrer NIRS-Spektren.
Das Ziel der vorliegenden Erfindung ist die Erhöhung der Genauigkeit und der Zuverlässigkeit sowie die Beschleunigung der Ermittlung des Gehalts von Gesamtfett, Protein, Trockenmilchsubstanz, Anzahl der Körperzellen, Bakterien und anderen Komponenten der Rohmilch. Das weitere Ziel gemäß einer der Ausführungsformen der Erfindung ist die Kostensenkung der Kalibrierung. Das technische Ergebnis der Anwendung des vorliegenden Verfahrens ermöglicht die Ermittlung der o. g. Milchkomponenten gemäß den geltenden "Technischen Vorschriften für Milch und Milchwaren" sowie GOST R 25054-2003 "Natürtiche Kuhmilch ― Rohstoffe", darunter auch unter Einsatz der NIRS Spektrometer, die in die Produktionslinien eingebaut werden.
Das gesetzte Ziel wird folgenderweise erreicht: Die Korrektur des Kalibrierungsmodells wird unter Berücksichtigung des Einflusses der dispersen Milchphasen und vor allem der Fettkügelchen auf die Spektren der milchbedingten Streuung der Verteilungsparameter je nach Teilchengröße vorgenommen; dafür werden Kalibrierchargen von Milch mit unkorrelierenden Variationen des Fettgehalts und der Verteilungsparameter je nach Größe der Fettkügelchen der Milch ausgesucht und/oder zusätzlich die Winkelverteilungen der Streuung der Proben im Kalibriersatz und der zu untersuchenden Milch gemessen, die Winkelverteilungen der Streuung auf der Basis der Kleinwinkelannäherung der Transporttheorie berechnet; die Werte der durchschnittlichen Kugelgrößen in den Proben werden nach der Anpassungsmethode der gemessenen Winkelverteilung an die Rechenwerte bewertet; das Kalibrierungsmodell wird unter Berücksichtigung der o. g. bewerteten Werte ermittelt und/oder die NIRS-Spektren der Zusammenwirkung des Kalibriersatzes der Milchproben für die vorgegebenen unkorrelierenden Variationen des Komponentengehalts und der Verteilung der Kugelgrößen berechnet, und anhand dieser Spektren wird das Kalibrierungsmodell ermittelt.

### Druckschriften - Literaturverzeichnis

G.V. Tverdokhleb, R.I. Ramanauskas Chemie und Physik von Milch und Milchwaren ― M.: DeLi Print, 2006. ― 360 S.
A.V. Kalinin, SPEKTROMETRISCHE SCHNELLANALYSE DER GÜTEZAHLEN VON MILCH UND MILCHGETRÄNKE, Patent RU 92191 vom 10.03.2009.
Tsenkova R., et al., Near Infrared Spectra of Cow's Milk for Milk Quality Evaluation: Disease Diagnostics and Pathogen Identification, J. of Near Infrared Spectr., 14, ...(2006)...,
Walstra, P. 1965. Light Scattering by Milk Fat Globules. Netherlands Milk Dairy J., 19:93
Anderson M. E., et al., Spectrophotometric method for simultaneously analyzing protein and fat contents in milk, US Patent 4497898, 1985.
Kalinin A.V., et al., "Effect of dispersion structure variation on chemometrical calibration of near-infrared spectrometer: protein fractions in milk and reversed micelles solutions", Chemometrics and Intell. Lab. Systems, 97, 33(2009)38.
R. Muniz et al., Comparison of principal component regression and partial least square methods in prediction of raw milk composition by VIS-NIR spectrometry. Application to development of on-line sensors for fat, protein and lactose contents, Proc.XIX IMEKO World Congress Fundamental and Applied Metrology, September 6-11, 2009, Lisbon, Portugal
Saranwong S. and Kawano S., Project of system for nondestructive near infrared research of chemical components and total bacteria counts of raw milk, J. Near Infrared Spectr., 16, 2DD8, 389-398
I.V.Yaroslavsky et al., Inverse hybrid technique for determining the optical properties of turbid media from integrating-sphere measurements, Applied Optics, 35, No.34, 6797(1996)6811
S.N.Thennadil and E.B.Martin Empirical preprocessing methods and their impact on NIR calibrations: a simulation study J. Chemometrics 2005; 19: 77―89
M. F. Ertugay, et al., Effect of Ultrasound Treatment on Milk Homogenisation and Particle Size Distribution of Fat Turk J Vet Anim Sci, 28 (2004) 303-308
Z. He, et al., Study on measurement of milk constituents by NIR analysis, Proc. SPIE, Vol. 5771, 189 (2005)203; doi:10.1117/12.634778
X.H.Hu, Patent US "Method and Apparatus for Spectrophotometric Characterisation of Turbid Materials", Patent Application Publication, Pub. No. US 2009/0103085 Al
Kalinin A.V., et al., Calibration models for multi-component quantitative analyses of dairy with the use of two portable NIR spectrometers, J. Near Infrared Spectr., 16, 343(2008)348
H.W. Jensen et al., Computing the Scattering Properties of Participating Media Using Lorenz-Mie Theory, Proc. SIGGRAPH, ...
R. Attaie and R. L. Richtert, Size Distribution of Fat Globules in Goat Milk, 2000, J Dairy Sci., 83:940-944;
M-C. Michalski, et al., The size of native milk fat globules affects physico-chemical and sensory properties of Camembert cheese, Lait, 83 (2003) 131―143;
A. Dukhin et al, Use of Ultrasound for Characterizing Dairy Products, J. of Dairy Science, 2005, 88 (4) 1320;
A.V. Kalinin, V.N. Krasheninnikov, J. Appl. Spectr., 75 (2008) 288 (Russische und englische Fassung).
N.G. Khlebtsov, Über die Rolle der mehrfachen Streuung bei spektralturbidimetrischen Forschungen der dispersen Systeme, Zeitschrift für angewandte Spektroskopie. 1984. T. 40. Heft Nr. 2. S. 320-325,
A. Ishimaru, Wave Propagation and Scattering in RandomMedia ∼Academic, New York, 1978, Vol. 1.
A.V. Kalinin, V.N. Krasheninnikov, Nahinfrarotspektroskopie und die Regressionsanalyse der Komponenten der physiologischen Flüssigkeiten (am Beispiel von Milch), gesammelte Werke der 4. Troitsker Konferenz "Medizinische Physik und Innovationen", Moskau, den 21.-24. Juni 2010.

## Patentansprüche

1. Nahinfrarotspektroskopieverfahren (NIRS-Verfahren) zur gleichzeitigen Ermittlung des Gehalts der Komponenten (Fett, Protein, Wasser, Körperzellen, Bakterien und anderer Komponenten) in Milch einschließlich
- der Suche nach einem Satz der Milchproben mit bekanntem Gehalt der zu ermittelnden Komponenten (dem sog. Kalibriersatz),
- der Speicherung der NIRS-Spektren der Wechselwirkung der o. g. Proben mit der darauf einfallenden Strahlung,
- der Ermittlung des Regressions-(Kalibrierungs)-Modells zwischen den gespeicherten (gesammelten) Spektren und den bekannten Gehaltswerten der zu ermittelnden Komponenten,
- der Messung der NIRS-Spektren der Wechselwirkung der Testproben,
- der Prüfung des o. g. Kalibrierungsmodells anhand dieser NIRS-Spektren der Proben,
- der Messung von NIRS-Spektren der Wechselwirkung der zu untersuchenden Proben und
- der Ermittlung des Komponentengehalts in der zu untersuchenden Milch nach dem Kalibrierungsmodell unter Berücksichtigung der Prüfungsergebnisse,
**dadurch gekennzeichnet,**
**dass** die Kalibriercharge von Milch mit unkorrelierenden Variationen des Fettgehalts und der größengerechten Verteilungsparameter der Fettkügelchen und anderer Milchteilchen ausgesucht und das Kalibrierungsmodell unter Beachtung der Variation der Verteilungsparameter je nach Teilchengröße der Milch ermittelt wird, um die Genauigkeit und die Zuverlässigkeit bei der Gehaltsermittlung von Milchkomponenten zu erhöhen;

2. Verfahren nach Anspruch 1,
**dadurch gekennzeichnet,**
**dass** die Korrektur des Kalibrierungsmodells unter Berücksichtigung des Einflusses der dispersen Milchphasen und vor allem der Fettkügelchen auf die Spektren der milchbedingten Streuung der Verteilungsparameter je nach Teilchengröße vorgenommen wird, um die Genauigkeit und die Zuverlässigkeit bei der Gehaltsermittlung von Komponenten zu erhöhen; dafür werden einige Fraktionen der Rohmilch separiert und teilweise homogenisiert, um die vorgegebenen Werte des Gehalts von Fett und anderer Komponenten sowie die Verteilungsparameter je nach Größe der Fettkügelchen zu erhalten; danach werden diese Fraktionen in berechneten Verhältnissen vermengt, um die Kalibrierchargen der Proben mit unkorrelierenden Variationen des Fettgehalts und der Verteilungsparameter je nach Größe der Fettkügelchen in der Milch zu erhalten, wobei die letzteren in den Mischungen nach einem bekannten Verfahren geprüft werden.

3. Verfahren nach Anspruch 1,
**dadurch gekennzeichnet,**
**dass**, um die Ermittlung zu beschleunigen, zusätzlich die Spektral- und Winkelverteilungen der Streuung der Proben aus dem Kalibriersatz und der zu untersuchenden Milch mit Hilfe des NIRS-Spektrometers gemessen werden, wobei das NIRS-Spektrometer modifiziert wurde, um den Messwinkel der Streustrahlung zu variieren,
**dass** die Spektral- und Winkelverteilungen der Streuung auf Grund der Kleinwinkelannäherung nach der Strahlungstransporttheorie berechnet werden, dass die durchschnittlichen Kugelgrößen in den Proben nach der Methode der Anpassung der gemessenen Winkelverteilung an die Rechenwerte bewertet werden und
**dass** das Kalibrierungsmodell unter Berücksichtigung der o. g. bewerteten Werte ermittelt wird.

4. Verfahren nach Anspruch 1,
**dadurch gekennzeichnet,**
**dass** das Signal des NIRS-Spektrometers nach den vorher gemessenen Rauschspektren und der Hardwarefunktion des Geräts und den berechneten Spektral- und Winkelverteilungen der Streuung und der Absorption im Kalibriersatz der Milchproben für die vorgegebenen unkorrelierenden Variationen des Komponentengehalts und der Verteilung der Kugeln je nach Größen berechnet wird und
**dass** danach das Kalibrierungsmodell ermittelt wird, um die Kalibrierungskosten zu vermindern.

## Geänderte Patentansprüche

### Geänderte Patentansprüche gemäss Regel 137(2) EPÜ.

**1.** Nahinfrarotspektroskopieverfahren (NIRS-Verfahren) zur gleichzeitigen Ermittlung des Gehalts der Komponenten (Fett, Protein, Wasser, Körperzellen, Bakterien und anderer Komponenten) in Milch einschließlich
- der Suche nach einem Satz der Milchproben mit bekanntem Gehalt der zu ermittelnden Komponenten (dem sog. Kalibriersatz),
- der Speicherung der NIRS-Spektren der Wechselwirkung der o. g. Proben mit der darauf einfallenden Strahlung,
- der Ermittlung des Regressions-(Kalibrierungs)-Modells zwischen den gespeicherten (gesammelten) Spektren und den bekannten Gehaltswerten der zu ermittelnden Komponenten,
- der Messung der NIRS-Spektren der Wechselwirkung der Testproben,
- der Prüfung des o. g. Kalibrierungsmodells anhand dieser NIRS-Spektren der Proben,
- der Messung von NIRS-Spektren der Wechselwirkung der zu untersuchenden Proben und
- der Ermittlung des Komponentengehalts in der zu untersuchenden Milch nach dem Kalibrierungsmodell unter Berücksichtigung der Prüfungsergebnisse,
**dadurch gekennzeichnet,**
**dass** die Kalibriercharge von Milch mit unkorrelierenden Variationen des Fettgehalts und der größengerechten Verteilungsparameter der Fettkügelchen und anderer Milchteilchen ausgesucht und das Kalibrierungsmodell unter Beachtung der Variation der Verteilungsparameter je nach Teilchengröße der Milch ermittelt wird, um die Genauigkeit und die Zuverlässigkeit bei der Gehaltsermittlung von Milchkomponenten zu erhöhen;

**2.** Verfahren nach Anspruch 1,
**dadurch gekennzeichnet,**
**dass** die Korrektur des Kalibrierungsmodells unter Berücksichtigung des Einflusses der dispersen Milchphasen und vor allem der Fettkügelchen auf die Spektren der milchbedingten Streuung der Verteilungsparameter je nach Teilchengröße vorgenommen wird, um die Genauigkeit und die Zuverlässigkeit bei der Gehaltsermittlung von Komponenten zu erhöhen; dafür werden einige Fraktionen der Rohmilch separiert und teilweise homogenisiert, um die vorgegebenen Werte des Gehalts von Fett und anderer Komponenten sowie die Verteilungsparameter je nach Größe der Fettkügelchen zu erhalten; danach werden diese Fraktionen in berechneten Verhältnissen vermengt, um die Kalibrierchargen der Proben mit unkorrelierenden Variationen des Fettgehalts und der Verteilungsparameter je nach Größe der Fettkügelchen in der Milch zu erhalten, wobei die letzteren in den Mischungen nach einem bekannten Verfahren geprüft werden.

**3.** Verfahren nach Anspruch 1,
**dadurch gekennzeichnet,**
**dass**, um die Ermittlung zu beschleunigen, zusätzlich die Spektral- und Winkelverteilungen der Streuung der Proben aus dem Kalibriersatz und der zu untersuchenden Milch mit Hilfe des NIRS-Spektrometers gemessen werden, wobei die Messungen beim Variieren des Messwinkels durchgeführt werden, dass die Spektral- und Winkelverteilungen der Streuung auf Grund der Kleinwinkelannäherung nach der Strahlungstransporttheorie berechnet werden, dass die durchschnittlichen Kugelgrößen in den Proben nach der Methode der Anpassung der gemessenen Winkelverteilung an die Rechenwerte bewertet werden und
**dass** das Kalibrierungsmodell unter Berücksichtigung der o. g. bewerteten Werte ermittelt wird.

**4.** Verfahren nach Anspruch 1,
**dadurch gekennzeichnet,**
**dass** das Signal des NIRS-Spektrometers nach den vorher gemessenen Rauschspektren und der Hardwarefunktion des Geräts und den berechneten Spektral- und Winkelverteilungen der Streuung und der Absorption im Kalibriersatz der Milchproben für die vorgegebenen unkorrelierenden Variationen des Komponentengehalts und der Verteilung der Kugeln je nach Größen berechnet wird und
**dass** danach das Kalibrierungsmodell ermittelt wird, um die Kalibrierungskosten zu vermindern.
